# EUROPEAN PATENT APPLICATION

(11) **EP 2 781 590 A1**
(43) Date of publication of application: **24.09.2014**
(21) Application number: 12848980.4
(22) Date of filing: 16.11.2012
(51) Int. Cl.: C12M 3/00, C12N 5/071, C12N 11/00

(54) **DEVICE FOR MANUFACTURING SUPPORTING BODY FOR CELL STRUCTURE MANUFACTURE**

(30) Priority: 18.11.2011 JP 2011252495
(71) Applicant: Saga University, Saga-shi, Saga 840-8502 (JP); Cyfuse Biomedical K. K., Bunkyo-ku Tokyo 1130033 (JP)
(72) Inventor: NAKAYAMA Koichi, Saga-shi Saga 840-8502 (JP)
(74) Representative: Denison, Christopher Marcus
(86) International application number: PCT/JP2012/079800
(87) International publication number: WO 2013/073672

(57) **Abstract**

A device for manufacturing a support for fixing cells of the present invention is capable of easily manufacturing a support that has needle-like bodies arranged on a base. The manufacturing device 2 is provided with a base 10, a holder 4, an insertion guide 6 and an alignment mechanism. The base 10 is retained by a holder 4. The base 10 is provided with a plurality of openings 15, into which needle-like bodies 20 are inserted and fixed. An insertion guide 6 is provided next to the holder 4 and includes slots 12 for placing a plurality of needle-like bodies 20 generally parallel to each other. The insertion guide 6 is provided with a plurality of slots 12 for placing the needle-like bodies 20 at corresponding positions to the openings 15, so that the alignment mechanism moves the insertion guide 6 such that the feed openings 12a are positioned adjacent to the openings 15. After positioning the feed openings 12a adjacent to the openings 15, the needle-like bodies 20 are introduced into the openings 15, whereby the needle-like bodies 20 are implanted into the base 10 to form a support.

## Description

### TECHNICAL FIELD

The present invention relates to a device for manufacturing a support used as a scaffold for producing a three-dimensional structure of cells.

### BACKGROUND ART

Recently, research for regenerative medicine is gradually making progress. For example, artificially cultured cells are used instead of the damaged cells in the affected area to regenerate the affected area for treatment. As a method for culturing cells for this purpose, for example, two-dimensional cultivation is conventionally well known in which cells are cultured on a Schale, or a petri dish. The cells formed by two-dimensional cultivation are peeled off from the Schale, or the petri dish, by a certain treatment and used in the affected area. For example, Patent Document 1 describes an improved method for generating chondroid tissues used for treating damage of a cartilage of a joint.

Furthermore, in order to enhance the therapeutic effect, techniques for constructing cells in three-dimensions for regenerative medicine have been studied and development thereof is steadily progressing. For example, as one procedure for constructing cells into a three-dimensional structure, a method that uses a support as a scaffold upon cultivation has been developed. Patent Documents 2 and 3 describe such supports as scaffolds.

Now, as a support used for producing a three-dimensional structure of cells, a support which has a plurality of needle-like bodies implanted into a base (substrate) has been developed as described, for example, in Patent Document 4, but manufacture of a support requires more time and trouble due to the increase in the number of the needle-like bodies to be implanted into the base.

### PRIOR ART DOCUMENT(S)

### PATENT DOCUMENT(S)

Patent Document 1: Japanese Patent Application Publication No. 2007-502127
Patent Document 2: Japanese Patent Application Publication No. 2004-216119
Patent Document 3: Japanese Patent Application Publication No. 2006-513013
Patent Document 4: International Publication No. 2008/123614

### SUMMARY OF THE INVENTION

### PROBLEM TO BE SOLVED BY THE INVENTION

The present invention was made under the above-described circumstances, and has an objective of facilitating manufacture of a support which has needle-like bodies arranged on a base.

### MEANS TO SOLVE THE PROBLEME

The present invention is as follows.
(1) A device for manufacturing a support for fixing cells, characterized by comprising:
   a base having arranged openings into which needle-like bodies are implanted;
   a holder for holding the base;
   an insertion guide having a plurality of slots for placing the needle-like bodies to correspond with the openings, where feed openings are formed at one ends of the slots for feeding the needle-like bodies into the base.
(2) The device according to (1), further comprising an alignment mechanism for moving the insertion guide so as to align the feed openings with the openings in the base held by the holder.
(3) The device according to (1), wherein the openings in the base are arranged in a matrix configuration.
(4) The device according to (1), characterized in that the alignment mechanism aligns the feed openings of the slots with the openings in the base by adjusting the position of the insertion guide.
(5) A method for manufacturing a support for fixing cells, characterized by comprising the steps of:
   setting needle-like bodies onto the insertion guide of the device according to any one of (1) to (4); and
   implanting the needle-like bodies into the base of the manufacturing device.

### EFFECT OF THE INVENTION

With a manufacturing device of the present invention, a support having a plurality of needle-like bodies implanted into a base can easily be manufactured by introducing the needle-like bodies into arranged openings formed in the base.

### BRIEF DESCRIPTION OF THE DRAWINGS

[Figure 1] Figure 1 is a perspective overview showing an appearance of a manufacturing device of the present invention.
[Figure 2] Figure 2 is a perspective overview of a manufacturing device of the present invention having a plurality of stacked bases set therein.
[Figure 3] Figure 3 is a perspective overview showing an embodiment of a manufacturing device of the present invention.
[Figure 4] Figure 4 is a perspective overview of a manufacturing device of the present invention having a plurality of stacked bases set therein.
[Figure 5] Figure 5 illustrates manufacture of a support by sequentially inserting the needle-like bodies from the pointed ends into the openings set in a matrix configuration, starting from the lower toward the upper rows of the openings.
[Figure 6] Figure 6 illustrates manufacture of a support by sequentially inserting the needle-like bodies from the back ends into the openings set in a matrix configuration, starting from the upper toward the lower rows of the openings.
[Figure 7] Figure 7 is a partial cross-sectional view for illustrating an insertion guide provided with a click mechanism and peripheral structures thereof.
[Figure 8] Figure 8 is a perspective view for illustrating a pushing member that is capable of collectively inserting the plurality of needle-like bodies into the openings.
[Figure 9] Figure 9 is a flowchart showing a procedure for manufacturing a support.
[Figure 10] Figure 10 is a perspective view of a support manufactured with a manufacturing device.
[Figure 11] Figure 11 is an illustration showing how to form a three-dimensional structure by building up spheroids on the manufactured support.
[Figure 12] Figure 12 is an illustration showing how to obtain a three-dimensional structure by pulling up the three-dimensional structure together with the sheet from the support.

### EMBODIMENTS FOR CARRYING OUT THE INVENTION

### [General Outline]

Conventionally, in order to produce a three-dimensional structure of cells by arranging cells in three dimensions, a support that serves as a scaffold is used. Manufacture of this support, however, requires trouble and time.

In particular, in a case where a three-dimensional structure of cells is produced by using a base (substrate) provided with needle-like bodies as a support where cell masses such as spheroids are pierced through these needle-like bodies, the support that serves as a scaffold may be formed by implanting a plurality of needle-like bodies into a base to obtain a larger three-dimensional structure. In order to provide a larger three-dimensional structure, however, a larger number of needle-like bodies needs to be implanted into the base and therefore complicates the formation of the support. In view of such problems, the present invention provides a device for facilitating manufacture of a support by simply implanting multiple needle-like bodies into a base (a device for manufacturing a support for fixing cells). The present inventor has invented a device for manufacturing a support wherein the device: holds a base having a plurality of openings for attaching needle-like bodies; is provided with an insertion guide for the needle-like bodies in a movable manner which can place the needle-like bodies at corresponding positions with respect to the plurality of openings formed in the base; and comprises a mechanism (alignment mechanism) for moving the insertion guide so as to allow introduction of the needle-like bodies into the openings in the base.

Hereinafter, specific embodiments of the manufacturing device of the present invention will be described. The following embodiments are merely examples for illustrating the present invention, and the present invention is not intended to be limited by these embodiments. The present invention can be carried out in various aspects without departing from the spirit thereof.

The present specification incorporates the content of the specification of Japanese Patent Application No. 2011-252495 (filed on November 18, 2011) based on which the present application claims priority. All of the publications such as technical documents, laid-out publications, patent publications and other patent documents cited herein are incorporated by reference herein in their entirety.

### [Embodiment (I)]

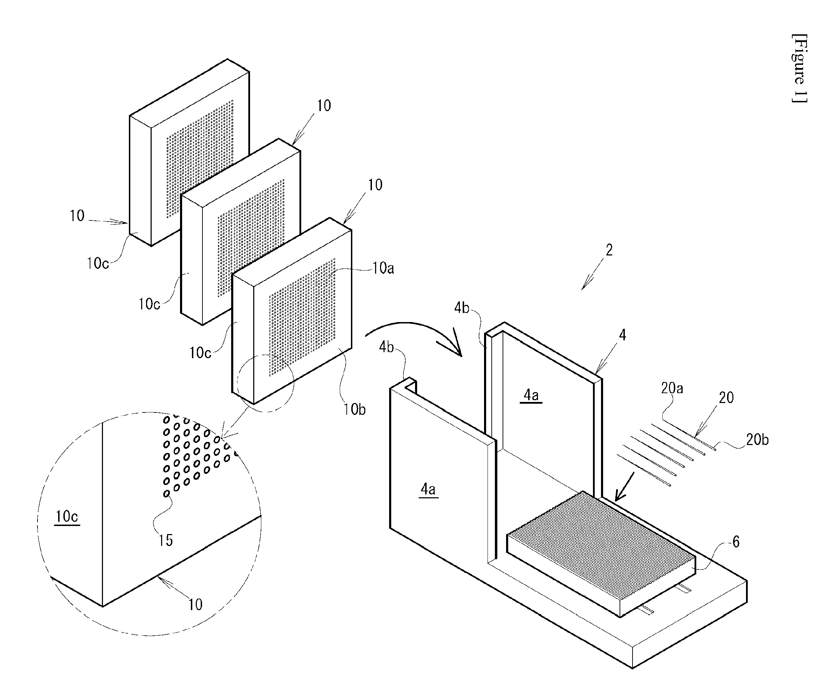

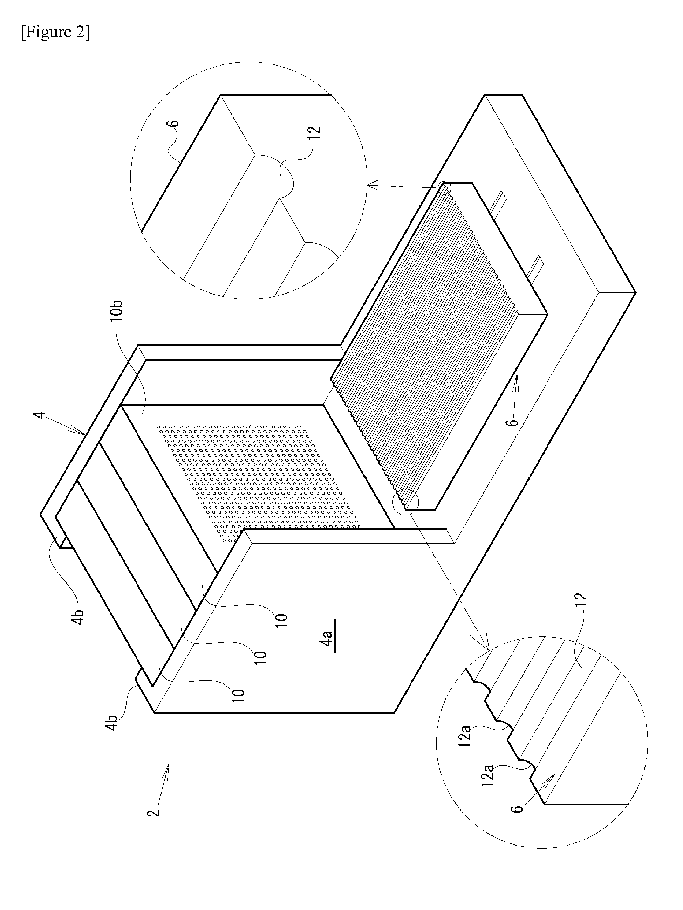

Figure 1 is an illustration for showing an overview of a manufacturing device of the present invention. Moreover, Figure 2 is a perspective view showing an overview of the manufacturing device shown in Figure 1 configured by setting of a plurality of bases.

As shown in Figures 1 and 2, the manufacturing device 2 is provided with a base 10, a holder 4 and an insertion guide 6, and optionally further provided with an alignment mechanism. The base 10 that serves as a foundation of the main part of the support may be formed, for example, into a plate. To hold this base 10, the holder 4 is provided with side walls 4a and stoppers 4b to match the shape of the base 10. The base 10 is formed with a plurality of openings 15 set in a matrix configuration, into which needle-like bodies 20 are inserted and secured. An insertion guide 6 provided next to the holder 4 has slots 12 for placing the plurality of needle-like bodies 20 generally parallel to each other. The needle-like body 20 is placed on each slot 12 such that the pointed end 20a, rather than the back end 20b, of the needle-like body 20 is positioned closer to the holder 4 side. As long as the needle-like bodies 20 serve as skewers, they are not limited to a needle shape with a sharp pointed end and a thicker back end. Accordingly, the shape of the needle-like bodies 20 may be formed such that, for example, the thickness is even from the pointed ends to the back ends, or the thickness has alternate peaks and recessed portions to have a shape of a screw. The material of the needle-like bodies 20 is not particularly limited, and may be formed by a resin such as polypropylene or nylon, or an alloy such as stainless steel. Although Figures 1 and 2 show an embodiment where the holder 4 holds three bases 10, the number of bases 10 held by the holder 4 is not necessarily limited to three and the number may be less than three or four or more.

At the end of each slot 12 of the insertion guide 6 on the holder 4 side, a feed opening 12a is formed. Through these feed openings 12a, the needle-like bodies 20 placed in the slots 12 can be fed outside the insertion guide 6, for example, to the base 10. An alignment mechanism provided in the manufacturing device 2 is capable of bringing the insertion guide 6 into contact with the base 10 held by the holder 4, and applying pressure in the direction toward the holder. For example, the plurality of slots 12 are formed in the insertion guide 6 to correspond with the matrix of openings 15 formed in the base. The slots may be formed into any shape as long as they place the needle-like bodies in a movable manner.

In an embodiment configured as a matrix, for example, of 26 rows by 26 columns (26 x 26) of openings 15 formed in the base 10, each row has 26 openings 15 formed while 26 slots 12 are formed in the insertion guide 6 to match all of the openings 15 in a row. According to the present invention, however, the number of slots 12 provided in the insertion guide 6 may be a part of the number of the openings 15. Each slot 12 can place the needle-like body 20 in a movable manner and has a feed opening 12a formed at the end thereof on the holder 4 side for feeding the needle-like body 20 outside the insertion guide 6.

The alignment mechanism positions these feed openings 12a adjacent to the openings 15 in the base 10 so that the needle-like bodies 20 placed on the slots 12 can be introduced from the slots 12 into the openings 15. A method for introducing the needle-like bodies into the openings, for example, includes, but is not limited to, a method of pushing out the needle-like bodies by application of force to the back ends 20b of the needle-like bodies in a direction toward the base using a pushing member described below, and an electric method utilizing electric charge. Any method can be employed as long as the needle-like bodies can be introduced into the openings. Although the feed openings 12a and the openings 15 in the base 10 are preferably set without a gap, the feed openings 12a and the openings 15 may be spaced apart to an extent that does not hinder the introduction of the needle-like bodies 20.

As the needle-like bodies are introduced into the base, the needle-like bodies penetrate through the openings 15 from the near side of the base along the hole formed in the base, and protrude out of the openings on the other side of the base, thereby being implanted into the base. In this case, when a plurality of bases is stacked, the needle-like bodies that have penetrated through the first base face the second base that is next in the array. However, since this second base also has openings formed in the same pattern as the first base, the needle-like bodies can be introduced into the openings 15 of the second base. Accordingly, even when a plurality of bases is stacked, the needle-like bodies do not get stuck and become bent by the second base.

When bases having different opening patterns are arrayed, there may be a problem that the needle-like bodies that have penetrated through the first base become stuck in the second base as described above. Therefore, when bases having different opening patterns are used, the base may be arrayed by leaving a space for the length of the needle-like bodies, or one base rather than a plurality of bases may be set into the holder so as to insert the needle-like bodies into this base for each respective operation.

For example, 26 x 26 openings 15 (the number of the openings 15 is not limited to 26 x 26) are formed in each base 10. The interval (pitch) between the adjacent openings 15 is suitably determined according to the size of the cell masses (spheroids) pierced into the needle-like bodies 20 upon producing a three-dimensional structure of cells. In accordance with this interval (pitch) between the openings, the interval between the needle-like bodies of the support manufactured by the manufacturing device of the present invention is defined. Although the interval between the needle-like bodies may vary depending on the size of the cell masses (spheroids) pierced therethrough, it is preferably defined to be generally 100% to 110% of the length for the diameter of the cell mass. In one embodiment, when the diameter of the cell mass is 1 mm, the interval between the needle-like bodies is preferably about 1 mm to 1.1 mm. The interval between the openings is set such that the needle-like bodies are aligned with spaces that allow contact with the adjacent cell masses when the cell masses are pierced through the needle-like bodies.

The support manufactured by implanting the needle-like bodies into the base may be drawn out in the longitudinal direction of the insertion guide, or taken out from above the insertion guide. In particular, when a plurality of bases are arranged and the needle-like bodies are inserting into the openings of the second base, the needle-like bodies may be drawn out in the longitudinal direction of the insertion guide away from the second base.

### [Embodiment (II)]

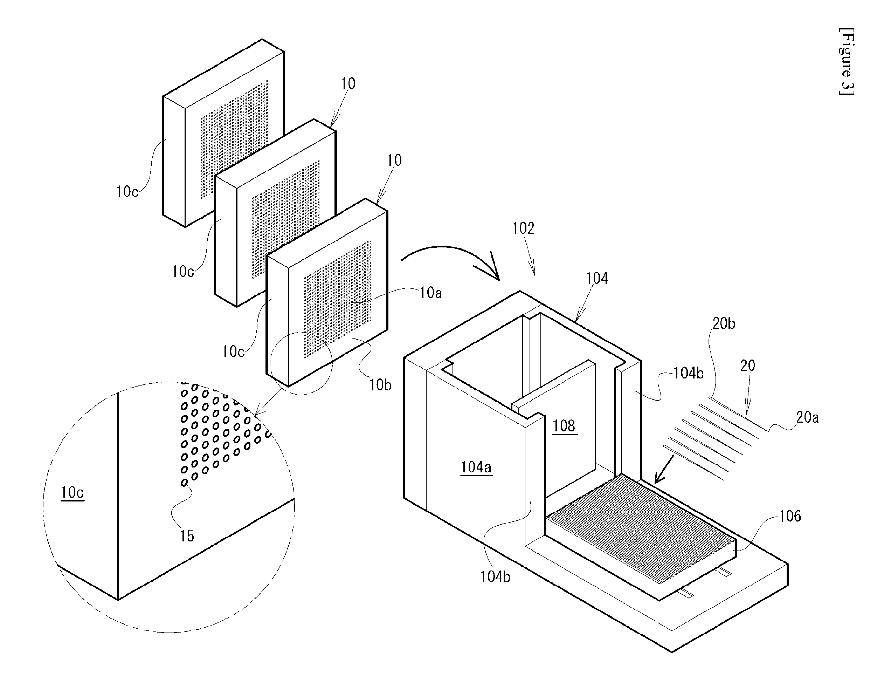

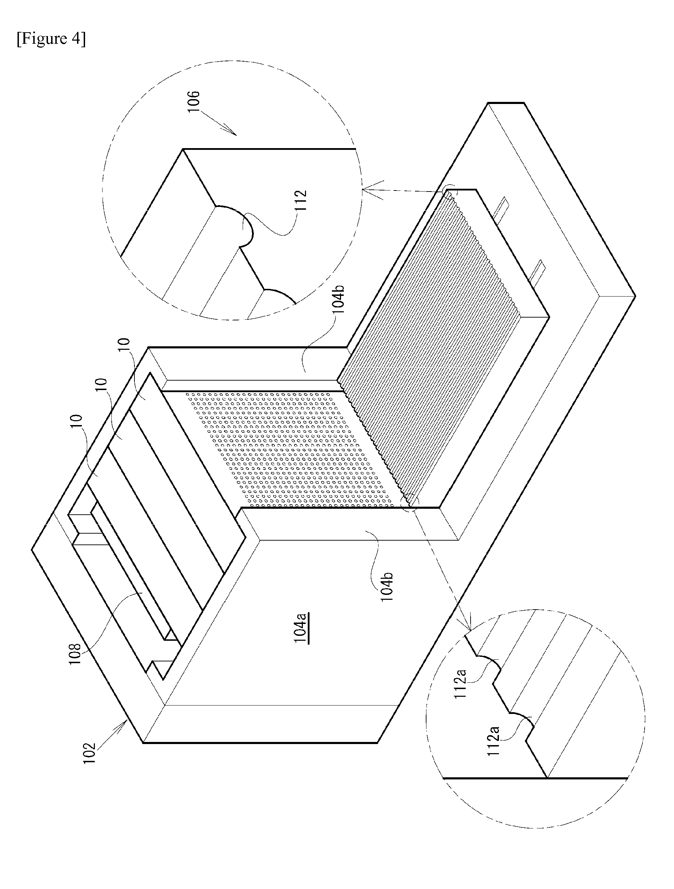

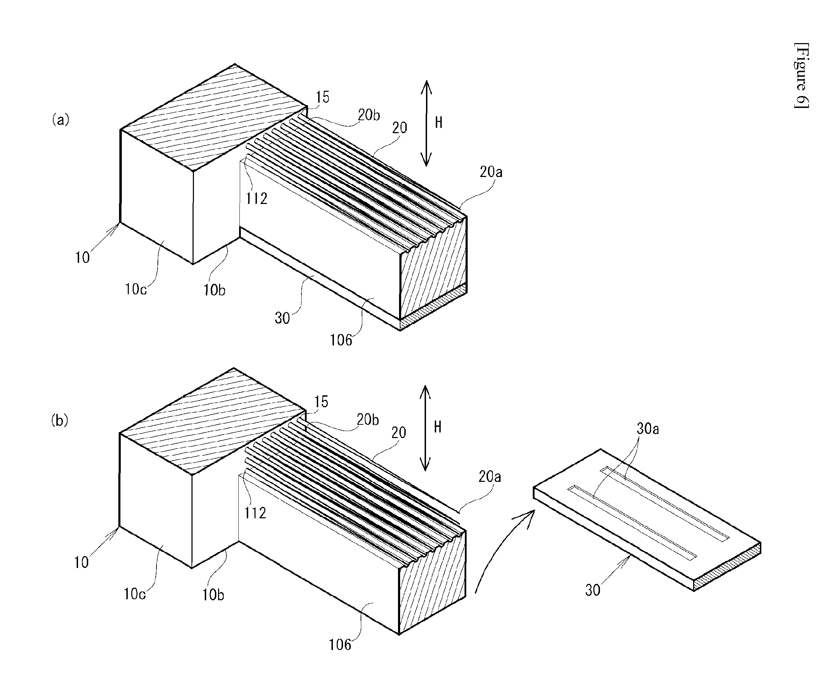

In the above-described manufacturing device 2, the needle-like bodies 20 are introduced into the openings 15 from the pointed ends 20a. The device may also be configured such that the needle-like bodies 20 are introduced into the openings 15 of the base 10 from the back ends 20b. In this case, the needle-like bodies 20 that are placed into the base 20 would protrude toward the insertion guide 6 side (see Figure 6). Therefore, the needle-like bodies 20 need to be introduced into the openings by moving the insertion guide 6 from top to bottom. Figure 3 is an illustration of a manufacturing device which introduces the needle-like bodies 20 into the openings 15 in the base 10 from the back ends 20b of the needle-like bodies 20. Furthermore, Figure 4 is a perspective overview showing the manufacturing device shown in Figure 3 holding a plurality of bases.

As shown in Figure 3 and 4, the manufacturing device 102 is provided with bases 10, a holder 104, an insertion guide 106, an alignment mechanism, a biasing board 108 and the like. The holder 104 has side walls 104a that make contact with the lateral surfaces 10c of the base 10, and stoppers 104b that make contact with the peripheral part 10b of the base 10 by being pressed by the peripheral part 10b. The biasing board 108 presses the bases 10 in the holder 104 toward the insertion guide 106 side such that the peripheral part 10b of the base 10 presses and makes contact with the stoppers 104b, whereby the base 10 approaches the insertion guide 106 side and is held thereby.

The manufacturing device 102 is provided with the biasing board 108, which biases the bases 10 in the holder 104 toward the insertion guide 106 side. As the biasing board 108 presses the bases 10 toward the insertion guide 106 side, the peripheral part 10b of the base 10 is securely fixed to the holder 104 with being pressed and contacted thereto to keep the base from moving. Thus, for example, once the base 10 having the needle-like bodies 20 completely implanted is drawn out from the holder 104, the base 10 adjacent to this drawn base 10 is pressed by the biasing board 108 and moves toward the insertion guide 106 side. Once the movement is initiated, the peripheral part 10b of the base 10 is pressed to and makes contact with the stoppers 104b of the holder 104 and halts there, the openings 15 in the base 10 are exposed to allow communication with the feed openings 112a of the insertion guide 106. Once the openings 15 are exposed, the needle-like bodies 20 are again placed in the slots 112 of the insertion guide 106 to be introduced from the slots 112 into the openings 15, thereby implanting the needle-like bodies 20 into the openings 15 of the base 10. Although three bases 10 are illustrated to be held in the holder 104 in Figure 3 and 4, the number of the bases 10 is not limited thereto and may be four or more or less than three.

### [Alignment mechanism]

The manufacturing device 2 shown in Figures 1 and 2 as well as the manufacturing device 102 shown in Figures 3 and 4 are each provided with an alignment mechanism for aligning the feed openings 12a, 112a of the slots 12, 112 with the openings 15 of the base 10. Examples of members that constitute the alignment mechanism include, but not limited to, a spacer or a click mechanism.

The manufacturing device 2 illustrated in Figures 1 and 2 is provided with an alignment mechanism which can adjust the position (e.g., height) of the insertion guide 6. By adjusting the height of the insertion guide 6, one can determine which opening 15 communicates with the feed opening 12a. Although no gap preferably exists between the feed openings 12a of the insertion guide 6 and the openings 15 in the base 10, the feed openings 12a and the openings 15 may be spaced apart to an extent that does not hinder the introduction of the needle-like bodies 20. Even if the insertion guide 6 and the base 10 are positioned in this manner, the needle-like bodies 20 can be introduced from the slots 12 into the openings.

Various structures can be contemplated as means for adjusting the height of the insertion guide 6. Herein, an embodiment where the height is adjusted by stacking spacers under the insertion guide 6 is illustrated, but the method for adjusting the height of the insertion guide 6 is not limited to the following examples.

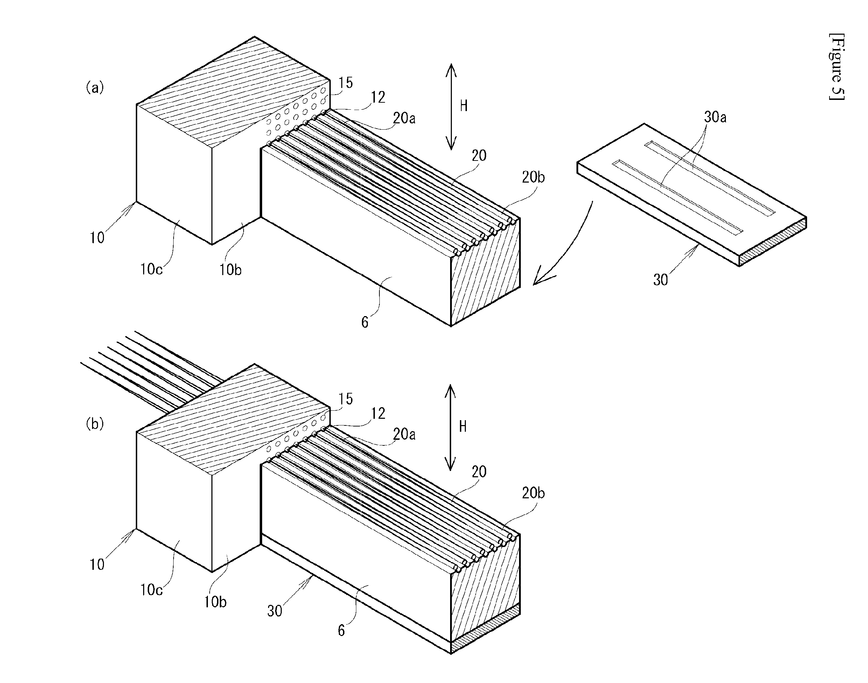

Figure 5 is an illustration for an alignment mechanism where the spacers are stacked under the insertion guide 6 to lift the insertion guide 6.

As shown in Figure 5(a), a spacer 30 is stacked and set under the insertion guide 6 so as to lift the insertion guide 6, thereby adjusting the position of the insertion guide 6 (Figure 5(b)). The thickness of the spacer 30 is made to equal, for example, the row pitch in the vertical direction H of the openings set as a matrix configuration so that the spacers 30 can be stacked to make the feed openings 12a of slots 12 in the insertion guide 6 to communicate with the openings 15 in the next upper row, thereby introducing the needle-like bodies 20 placed in the slots 12 into the openings 15. Once the needle-like bodies 20 are inserted from the pointed ends 20a into the openings 15 in a lower row, a spacer 30 may be stacked under the insertion guide 6 so that the feed openings 12a of the slots 12 can line up with the openings 15 in the next upper line, thereby allowing introduction of the needle-like bodies 20 into these openings 15.

Introduction of the needle-like bodies 20 can start from the openings (end openings) 15 in the bottommost row among the openings 15 set as a matrix configuration and the above-described operation is repeated so that the needle-like bodies 20 can be implanted into all of the openings 15 set as a matrix configuration. Moreover, depending on the support of interest and the shape of the three-dimensional structure of cells produced by said support, the needle-like bodies may not be implanted into all of the openings 15. Openings that are to be provided with the needle-like bodies can be selected among the openings set as as a matrix configuration so as to obtain a support with a desired shape, which can be used to obtain a three-dimensional structure of cells having a desired shape.

According to an embodiment where the needle-like bodies 20 are implanted into the base 10 by introducing and pushing the needle-like bodies 20 into the openings 15 from the pointed ends 20a until the back ends 20b are housed inside the openings 15 as shown in Figure 5, the insertion guide 6 can be moved from bottom to top along the vertical direction H to introduce the needle-like bodies 20 into the openings 15. The mechanism shown in Figure 5, however, may also introduce the needle-like bodies 20 into all of the openings 15 by moving the insertion guide 6 from top to bottom. In the case where the insertion guide 6 is moved from top to bottom, the insertion guide 6 is placed on spacers 30 stacked in advance so that the needle-like bodies 20 can be introduced into the openings 15 and implanted in the base 10 by moving the insertion guide 6 from top to bottom by removing the spacer 30 one by one along with the introduction of needle-like bodies 20 into the openings 15.

According to an embodiment where the height of the insertion guide 6 is adjusted by using the spacer 30 shown in Figure 5,recessed portions/projections may be provided on the upper surface of the spacer 30 while projections/recessed portions that fit the recessed portions/projections on the upper surface of other spacer 30 are provided on the back surface of the spacer 30, so as to prevent the spacers 30 from wobbling, thereby enhancing stability. For example, recessed portions 30a are provided on the upper surface of the spacer 30 so that these recessed portions 30a fit the projections (not shown) formed on the back surface of the insertion guide 6 to stably position the insertion guide 6 on the manufacturing device 2 and thereby keep the guide from moving. Since the insertion guide 6 can be held on the manufacturing device 2 without wobbling, stable communication between the feed openings 12a formed at one ends of the slots 12 and the openings 15 in the base 10 can be maintained and thus guidance of the needle-like bodies 20 placed in the slots 12 into the openings 15 can be ensured.

The alignment mechanism shown in Figures 5(a) and (b) may move the insertion guide 6 from bottom to top along the vertical direction H to implant the needle-like bodies 20 in its base 10, or may move the insertion guide 6 from top to bottom to implant the needle-like bodies 20 into the base 10. With another alignment mechanism shown in Figure 6 which will be described below, the insertion guide 6 is moved from top to bottom to implant the needle-like bodies 20 into the base 10.

Figure 6 is an illustration showing a part of the alignment mechanism in which the spacers 30 are stacked in advance under the insertion guide 106 shown in Figure 3 and 4 so that the needle-like bodies are sequentially introduced from top to bottom rows.

For example, after the needle-like bodies 20 are introduced into the openings (end openings) 15 in the uppermost row as shown in Figure 6(a), one spacer 30 is removed so as to lower the height of the insertion guide 106 by a row, thereby enabling the needle-like bodies 20 to be introduced into the openings 15 in the second row from the top. Once the needle-like bodies 20 are introduced into the openings 15 in the second row from the top, a spacer 30 is further removed as shown in Figure 6(b) to further lower the height of the insertion guide 106 by a row, thereby enabling the needle-like bodies 20 to be introduced into the openings 15 in the third row from the top. In this manner, the needle-like bodies 20 can be implanted into the base 10 by moving the movement guide 106 from top to bottom by stacking the spacers 30 in advance.

According to such an embodiment where the needle-like bodies 20 are introduced into the openings 15 from the back ends 20b and implanted into the base 10, the needle-like bodies 20 protrude out from the base 10 toward the insertion guide 106 side. Therefore, the insertion guide 106 is moved from top to bottom to introduce the needle-like bodies 20 into the openings 15.

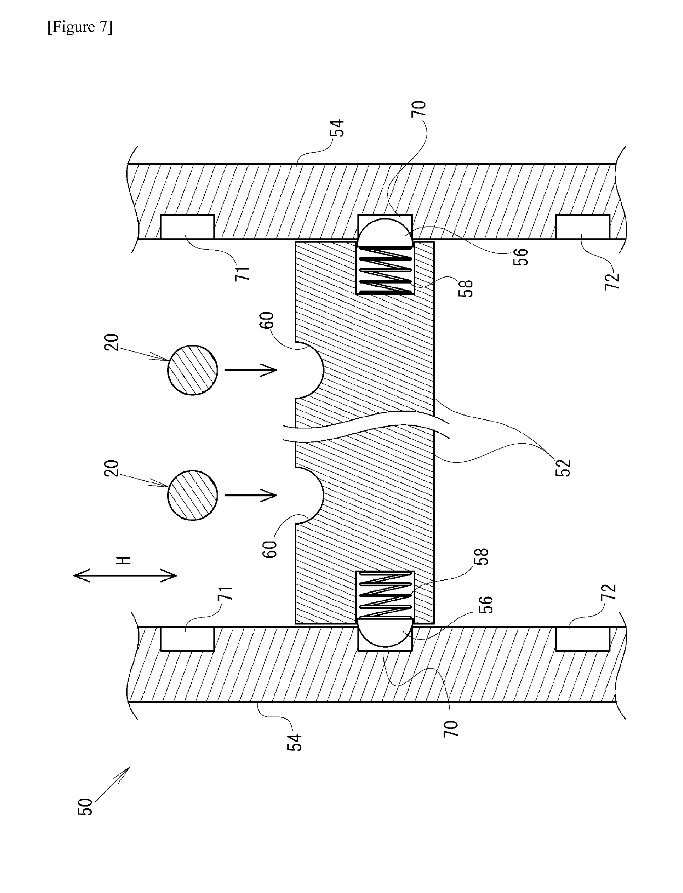

Furthermore, although spacers are used to lift/lower the insertion guides 6 and 106 in Figures 5 and 6 described above, embodiments for lifting/lowering the insertion guides in which the needle-like bodies 20 are placed are not limited thereto. For example, both sides of the insertion guide may be supported with the click mechanism so that the guide can be freely lifted/lowered. Lift or lowering of the insertion guide with the support of the click mechanism facilitates movement of the insertion guide. Figure 7 is a partial cross-sectional view cut across the alignment mechanism generally parallel to the vertical direction H, where the insertion guide is held by the click mechanisms to allow movement in the vertical direction H.

As shown in Figure 7, the click mechanism may be a generally used mechanism. For example, a click mechanism 50 comprises an insertion guide 52, and a guide support 54 that supports the insertion guide 52 movable in the vertical direction H. The insertion guide 52 is provided with slots 60 in which the needle-like bodies 20 are placed, engageable hooks 56, and springs 58 that bias the engageable hooks 56 outwardly. The guide support 54 has notches 70, 71 and 72 which can engage with the engageable hook 56.

In one embodiment, each of the notches 70 to 72 is formed along the vertical direction H with the intervals between the notches 70, 71 and 72 to align with the row pitch of the openings 15 in the base 10 (see Figure 1). Accordingly, for example, when the insertion guide 52 is moved upwardly such that the engageable hooks 56 engaged with the notches 70 engage with the notches 71, the insertion guide is aligned such that the needle-like bodies 20 can be introduced into the openings 15 in the next upper row. Furthermore, when the insertion guide 52 moves downwardly as the engageable hooks 56 engaged with the notches 70 engage with the notches 72, the insertion guide is aligned to allow introduction of the needle-like bodies 20 into the openings 15 in the next lower row. Since the notches 70 to 72 of the guide supports 54 are formed to match the row pitch of the openings 15 in the base 10, the click mechanisms can secure the insertion guide 52 in a stationary configuration such that the feed openings of the slots 60 can communicate with the following openings 15 of the base 10. Thus, use of the click mechanism, which has the insertion guide 52 provided with engageable hooks 56 and which has the guide supports 54 with notches 70 to 72 for supporting the insertion guide 52 movable in the vertical direction H, facilitates adjustment of the height of the insertion guide 52 without using the spacer 30 described above. As described above, the click mechanism may also be used as a means for adjusting the movement of the insertion guide.

Other than the above-described spacers or click mechanism, the insertion guide can also be aligned with a robot or by providing a height controlling mechanism.

Although in the above-described embodiment, the needle-like bodies are implanted in the vertical direction H, it should be understood that the present invention also contemplates a manufacturing device having a constitution that implants the needle-like bodies in a horizontal direction or a manufacturing device having a constitution that implants the needle-like bodies in an oblique direction.

### [Improvement in efficiency for introducing needle-like bodies]

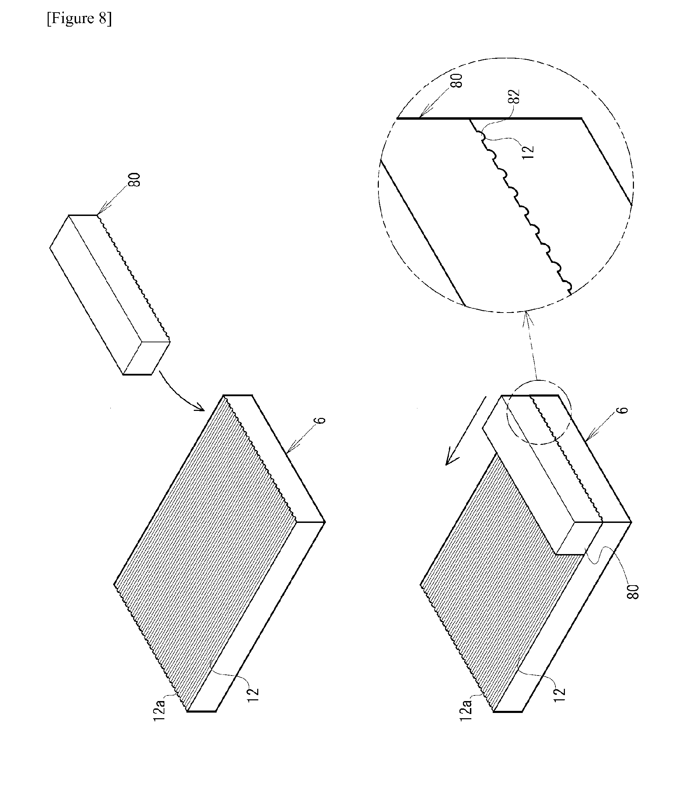

In the manufacturing device 2 or 102 illustrated in Figures 1 or 3, respectively, the needle-like bodies 20 may be manually introduced one by one from slots 12 or 112 of the insertion guide 6 or 106 into the openings 15 in the base 10, or a plurality of needle-like bodies 20 may collectively be introduced from the slots into the openings as shown in Figure 8.

Figure 8 is an illustration for an embodiment in which the needle-like bodies 20 placed in the slots 12 exemplified in Figure 1 or 2 are introduced into the openings 15 by using a pushing member which collectively pushes the needle-like bodies 20 into the openings 15 in the base 10. As shown in Figure 8, a plurality of hedges 82 that match the recessed portion of the slots 12 is formed in the pushing member 80, and the ends of the needle-like bodies 20 make contact therewith by being pushed with these hedges 82. In this state, as the pushing member 80 is moved toward the feed openings 12a, the needle-like bodies 20 placed on the slots 12 are pushed toward the base 10 and are introduced into the openings in the base 10 via the feed openings 12a. By forming the hedges 82 in the pushing member 80 for engaging with all of the slots 12, the needle-like bodies 20 in all of the slots 12 can collectively be introduced from the slots 12 into the base 10, thereby reducing the labor required to introduce the needle-like bodies 20 into the openings 15 and enhancing convenience.

### [Three-dimensional structure]

A three-dimensional structure of cells can be formed by piercing and building up aggregates of cells (spheroids) into the needle-like bodies 20 implanted in the base 10 with the manufacturing device 2, 102 of the present invention. Examples of cells used in this regard include undifferentiated cells or differentiated cells thereof, such as stem cells (ES cells, umbilical cord-derived cells, undifferentiated mesenchymal cells, etc.), somatic cells and tumor cells. In addition, fibroblasts, stem cells, vascular endothelial cells, epidermal cells, epithelial cells, osteoblasts, cartilage cells or adipose cells which can readily be induced to differentiate from undifferentiated mesenchymal stem cells can also be used, or cells such as articular cartilage cells or osteocytes may also be used. Moreover, the spheroids are not necessarily formed as an aggregate made of a single type of cells, but may be formed from multiple types of cells as long as spheroids can be formed.

Cells can broadly be classified into floating cells and scaffold-dependent cells, where the cells of blood system and immune system belong to the former and the cells of skin and bone belong to the latter. Cells such as skin or bone cells die in a floating state in a culture fluid, and thus need to be applied onto Schale such as glass for proliferation. Therefore, for example, when cells are collected in a culture vessel having a polytetrafluoroethylene-treated surface, the cells adhere each other in want of a scaffold, thereby forming cell aggregates, i.e., spheroids. Furthermore, if the spheroids adhere or fuse to each other, a larger configuration will result. By the intermediary of spheroids, the cells are considered to enter the quiescent phase of the cell cycle, thereby increasing protein production. Hence, according to the present invention, in order to induce the cells to enter the quiescent phase, the cells are made into spheroids and then formed into a three-dimensional structure of cells.

The culture fluid used for cell cultivation may be a commonly used synthetic or natural medium depending on the cells to be cultured. A synthetic medium is preferable considering bacterial/viral infection caused by an animal-derived substance, time of supply and quality stability. As a synthetic medium, for example, α-MEM (Minimum Essential Medium), DMEM (Dulbecco's modified Eagle medium), RPMI 1640 medium, CMRC medium, HAM medium, DME/F12 medium, MCDB medium or the like may be used. To these media, a physiologically active substance such as a proliferative factor, a growth factor or a hormone, or other various substances having a pharmacological action may appropriately be added. Addition of such substances may impart a particular function to the cultured cells or alter the original function of the cells. Examples of growth factors or cell proliferative factors include bone morphogenetic protein (BMP), fibroblast growth factor (FGF), transforming growth factor, insulin-like growth factor (IGF), platelet-derived growth factor (PDGF), vascular endothelial growth factor (VEGF), known serum components such as transferrin (concentration appropriately adjusted), and various vitamins and antibiotics such as streptomycin. Examples of hormones include insulin, transferrin, dexamethasone, hydrocortisone, thyroxine, 3,3',5-triiodothyronine, 1-methyl-3-butylxanthine and progesterone.

Other typical physiologically active substances typically include ascorbic acid (in particular, L-ascorbic acid), biotin, calcium pantothenate, ascorbic acid-2-phosphate, vitamins such as vitamin D, serum albumin, proteins such as transferrin, lipids, fatty acid source, linoleic acid, cholesterol, pyruvic acid, nucleosides for DNA and RNA synthesis, glucocorticoid, retinoic acid, glycerophosphate and monothioglycerol.

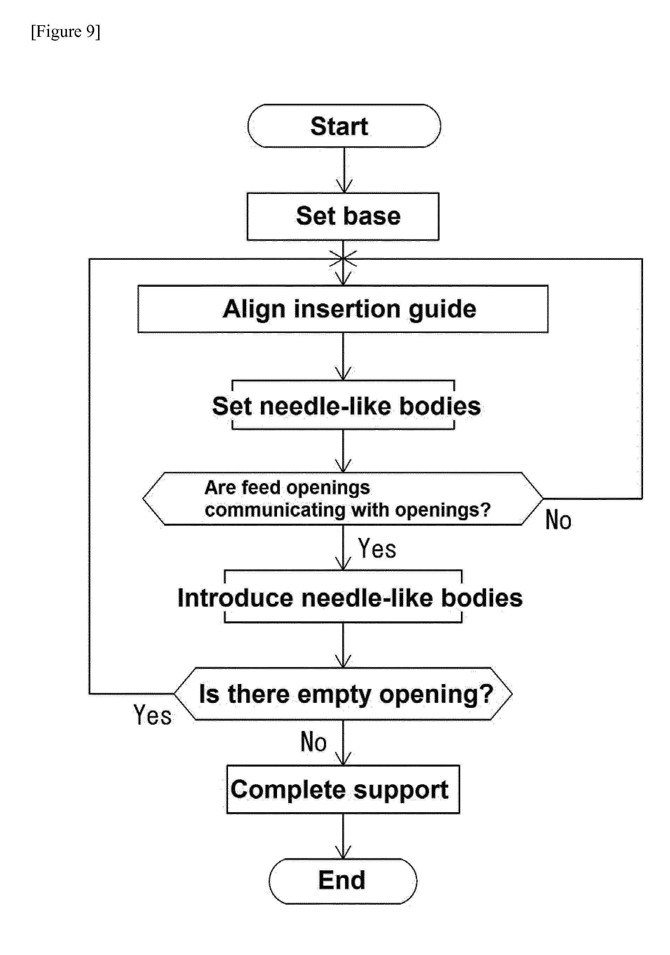

Figure 9 is a flowchart showing a typical procedure for manufacturing a support with a manufacturing device of the present invention. Herein, explanation is given with respect to the manufacturing device 2 illustrated in Figures 1 and 2 (hence, the alignment mechanism illustrated in Figure 5 is used).

As shown in Figure 9, the user sets a base 10 that suits a shape of a support to be manufactured into the holder 4. A plurality of slots 12 is formed in the insertion guide 6. Once the base 10 is set into the holder 4, alignment is performed, for example, by pressing and bringing the insertion guide 6 to make contact with the holder 4 such that the feed openings 12a of the slots 12 communicate with the openings (end openings) 15 in the bottommost row of the base 10. Once the insertion guide 6 is aligned such that the slots 12 of the insertion guide 6 communicate with the openings 15 in the base 10, the needle-like bodies 20 are placed in the respective slots 12. After the needle-like bodies 20 are placed in the respective slots 12, the needle-like bodies 20 placed in the slots 12 are moved and introduced into the openings 15.

Once the back ends 20b of all of the needle-like bodies 20 placed in the slots 12 are introduced into the openings 15, a spacer 30 is stacked under the insertion guide 6 to align the feed openings 12a of the slots 12 with the openings 15 in the next upper row. Following alignment of the insertion guide 6, needle-like bodies 20 are newly placed in the respective slots 12 to introduce the needle-like bodies 20 from the respective slots 12 into the openings 15. By repeating this operation, a support is completed which has a matrix of needle-like bodies 20 implanted into the base 10.

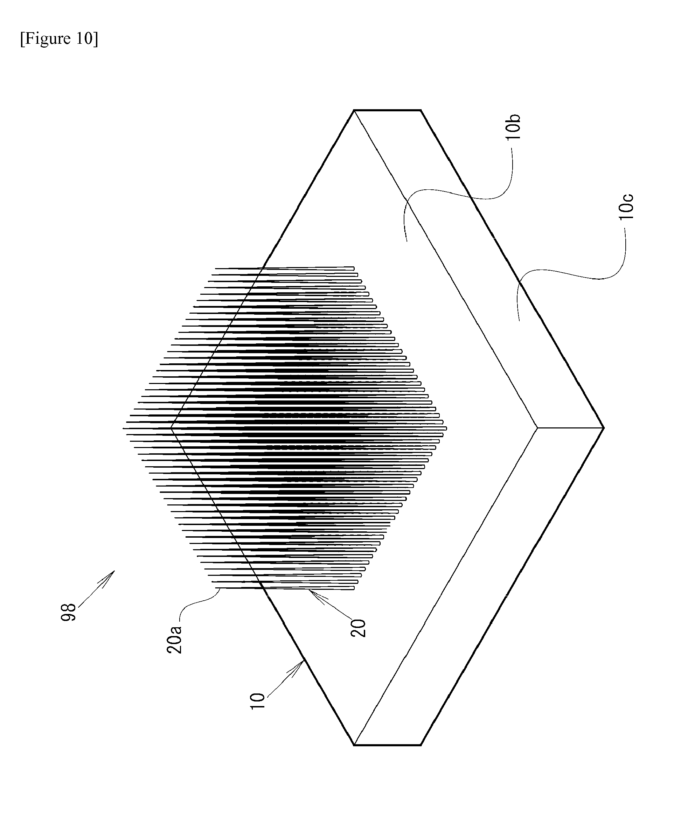

Figure 10 is a perspective view showing an appearance of a support manufactured with the manufacturing device.

As shown in Figure 10, after manufacturing the support 98 by implanting the needle-like bodies 20 into all of the openings 15 in the base 10, this support 98 is removed from the holder 4. When a plurality of bases 10 is held in the holder 4, the openings 15 of the following base 10 are exposed so that manufacture of the next support can immediately take place. The step of setting the base, the step of aligning the insertion guide, and the step of setting the needle-like bodies shown in Figure 9 are not necessarily carried out in the above-described order. For example, the insertion guide may be aligned after setting the needle-like bodies; or the insertion guide and the needle-like bodies may be aligned and be set at the same time. Although a total of 676 "26 x 26" needle-like bodies 20 are implanted into the base 10 in Figure 10, this is merely one example and the number of the needle-like bodies 20 may be larger or smaller than this number. The number and the length of the needle-like bodies implanted into the base may appropriately be selected to suit the shape of the three-dimensional structure of cells of interest.

### [Construction of three-dimensional structure]

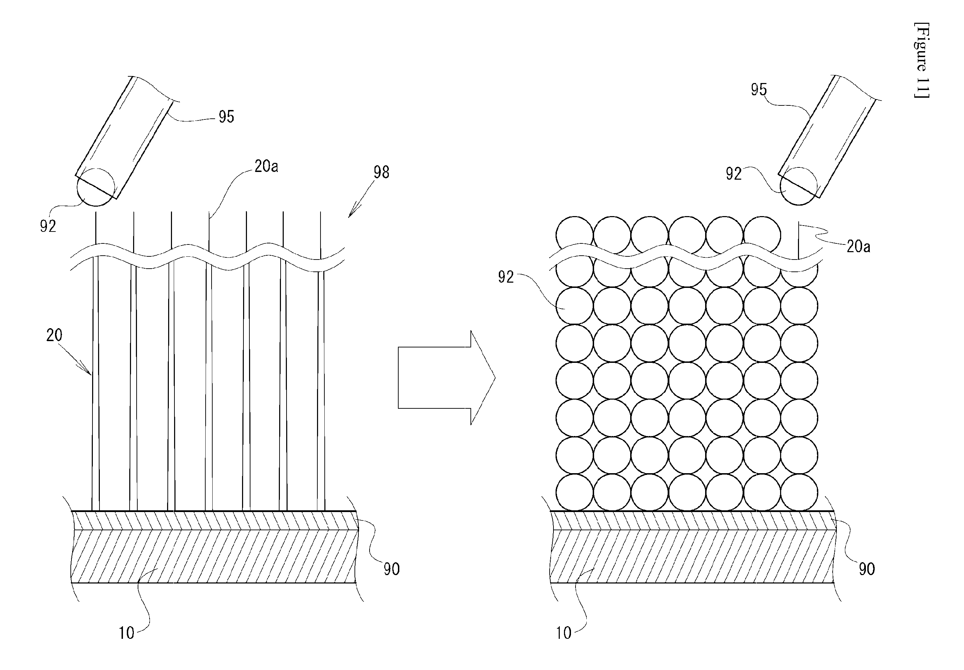

When a three-dimensional structure of cells is constructed with the support 98, for example, the support 98 is covered with a peelable sheet. The needle-like bodies 20 are pierced through this sheet and spheroids, in turn, are pierced through these needle-like bodies. Figure 11 is an illustration of how to form a three-dimensional structure of cells using the support 98 manufactured with the manufacturing device of the present invention.

The support 98 is covered with a sheet 90 as shown in Figure 11. The sheet 90 may be peeled off from the base 10, and, it may be formed, for example, to have a net-like pattern so that the needle-like bodies 20 can readily be pierced therethrough. Alternatively, the sheet may have pores that correspond to the needle-like bodies 20. By positioning this sheet 90 between the base 10 and the spheroids 92, a three-dimensional structure of cells formed based on the spheroids 92 can easily be removed from the base 10. Preferably, the sheet material is processed, for example, with fluorine or polyhydroxyethyl methacrylate polymer. Additionally, the sheet material may be Teflon (registered trademark), a resin such as poly-HEMA, an acrylic board, a vinyl chloride board, an ABS resin board, a polyester-based resin board or a polycarbonate board, or engineering plastics such as PP (polypropylene), ABS (acrylonitrile butadiene styrene), PE (polyethylene), POM (polyacetal), PC (polycarbonate), PEEK (polyether ether ketone), MCN (monomer casting nylon), 6N (6 nylon), 66N (66 nylon).

After covering the base 10 with the sheet 90, spheroids 92 are pierced and built up through the respective needle-like bodies 20 using a pipette 95 or the like. An operation to pierce the spheroids through the needle-like bodies may be performed not only with the pipette 95 but also with a robot arm, tweezers or the like, which may appropriately be selected by the user to pierce the spheroids through the needle-like bodies. Due to contact between the spheroids, the spheroids may fuse with each other in the axial direction (longitudinal direction) of the needle-like bodies.

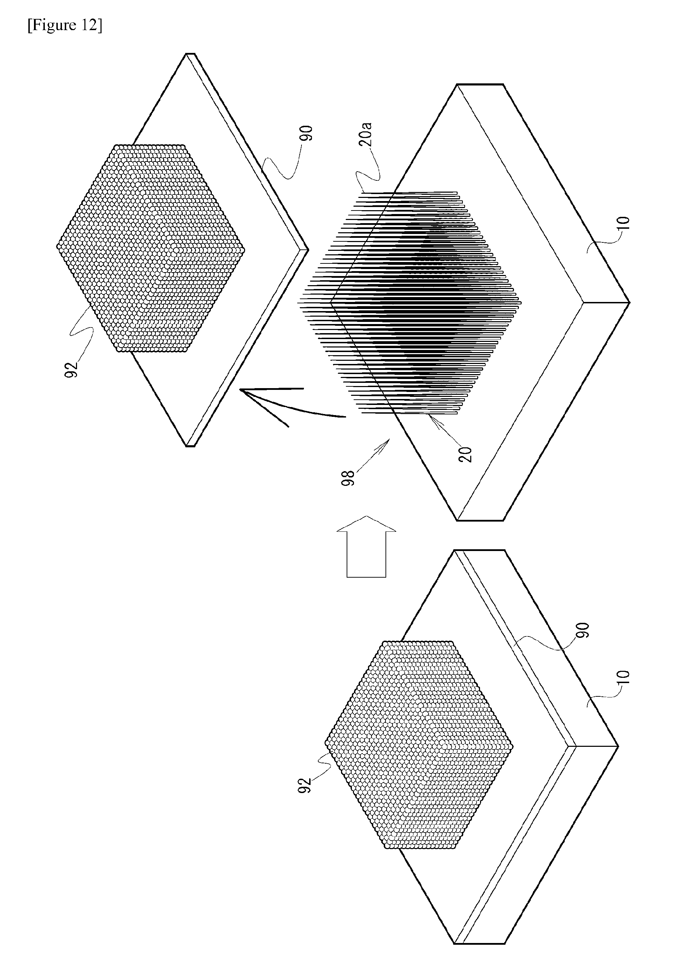

Moreover, since the needle-like bodies are aligned at an interval that allows contact between the adjacent pierced spheroids, the spheroids may also fuse with each other in the horizontal direction (transverse direction) with respect to the axes of the needle-like bodies. Due to such fusion of the spheroids, a three-dimensional structure of cells can be formed from spheroids 92 that are pierced through the needle-like bodies 20 on the base 10. Figure 12 is an illustration showing how to construct a three-dimensional structure by building up spheroids on a support and obtain this three-dimensional structure by removing it from the support together with the sheet after the adjacent spheroids have adhered to each other. As shown in Figure 12, after the adjacent spheroids 92 have adhered to each other, the sheet 90 is removed from the support 98 together with the three-dimensional structure of cells. Accordingly, a three-dimensional structure of cells formed based on spheroids can be obtained.

As described above, a manufacturing device of the present invention comprises a base holding member for holding a base, an insertion guide provided with slots for placing needle-like bodies, and a position adjusting member for adjusting the position of the insertion guide with respect to the substrate such that the slots of the insertion guide communicates with the openings in the base. As a result, the needle-like bodies can be implanted into the openings in the base row by row, thereby easily manufacturing a support for forming a three-dimensional structure of cells.

### DESCRIPTION OF SIGN(S)

- 2: Manufacturing device
- 4: Holder
- 4a: Side wall
- 4b: Stopper
- 6: Insertion guide
- 10: Base
- 12: Slots
- 12a: Feed openings
- 15: Openings
- 20: Needle-like bodies
- 20a: Pointed ends
- 20b: Back ends
- 30: Spacer
- 50: Click mechanism
- 52: Insertion guide
- 54: Guide support
- 56: Engageable hook
- 58: Spring
- 60: Slots
- 70, 71, 72: Notches
- 80: Pushing member
- 90: Sheet
- 92: Spheroids
- 95: Pipette
- 98: Supporting body
- 102: Manufacturing device
- 104: Holder
- 106: Insertion guide
- 108: Biasing board
- 112: Slots
- H: Vertical Direction

## Claims

1. A device for manufacturing a support for fixing cells, **characterized by** comprising:
a base having arranged openings into which needle-like bodies are implanted;
a holder for holding the base;
an insertion guide having a plurality of slots for placing the needle-like bodies to correspond with the openings, where feed openings are formed at one ends of the slots for feeding the needle-like bodies into the base.

2. The device according to Claim 1, further comprising an alignment mechanism for moving the insertion guide so as to align the feed openings with the openings in the base held by the holder.

3. The device according to Claim 1, wherein the openings in the base are arranged in a matrix configuration.

4. The device according to Claim 1, **characterized in that** the alignment mechanism aligns the feed openings of the slots with the openings in the base by adjusting the position of the insertion guide.

5. A method for manufacturing a support for fixing cells, **characterized by** comprising the steps of:
setting needle-like bodies onto the insertion guide of the device according to any one of Claims 1 to 4; and
implanting the needle-like bodies into the base of the manufacturing device.
